(19) **Europäisches Patentamt / European Patent Office / Office européen des brevets**

(11) **EP 2 185 133 B1**

(12) **EUROPEAN PATENT SPECIFICATION**

(45) Date of publication and mention of the grant of the patent:
**07.01.2015 Bulletin 2015/02**

(21) Application number: **08830894.5**

(22) Date of filing: **10.09.2008**

(51) Int Cl.:
*A61K 9/16* (2006.01)          *A61K 9/50* (2006.01)
*A61K 31/74* (2006.01)        *A61K 31/67* (2006.01)
*A61K 9/14* (2006.01)          *A61P 19/10* (2006.01)
*A61K 31/663* (2006.01)      *A61K 31/675* (2006.01)

(86) International application number:
**PCT/KR2008/005351**

(87) International publication number:
**WO 2009/035265 (19.03.2009 Gazette 2009/12)**

(54) **CALCIUM PHOSPHATE MICROSPHERES DRUG DELIVERY SYSTEM AND PROCESS FOR PREPARING THE SAME**

SYSTEM ZUR AUSGABE VON HEILMITTELN IN FORM VON CALCIUMPHOSPHAT-MIKROKUGELN UND VERFAHREN ZU SEINER HERSTELLUNG

SYSTÈME D'ADMINISTRATION DE MÉDICAMENTS SOUS FORME DE MICROSPHÈRES DE PHOSPHATE DE CALCIUM ET PROCÉDÉ DE PRÉPARATION DE CE SYSTÈME

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **10.09.2007 KR 20070091639**

(43) Date of publication of application:
**19.05.2010 Bulletin 2010/20**

(73) Proprietor: **Hans Biomed.Cor
Yuseong-gu
Daejeon 305-812 (KR)**

(72) Inventors:
• **LEE, Sang Cheon
Suwon-si
Gyeonggi-do 443-726 (KR)**
• **KIM, Kyung Ja
Gunpo-si
Gyeonggi-do 435-040 (KR)**
• **KIM, Chan Woo
Seoul 110-530 (KR)**
• **KIM, Sung Eun
Seoul 135-080 (KR)**
• **HWANG, Ho-Chan
Seoul 143-200 (KR)**

• **KANG, Ke-Won
Daejeon 305-370 (KR)**
• **CHAE, Ji-Hwa
Daejeon 305-345 (KR)**
• **SEO, Seog-Jin
Daejeon 302-844 (KR)**
• **HEO, Jae-Won
Daejeon 306-767 (KR)**

(74) Representative: **ABG Patentes, S.L.
Avenida de Burgos, 16D
Edificio Euromor
28036 Madrid (ES)**

(56) References cited:
**US-A1- 2002 155 144     US-A1- 2004 180 091
US-A1- 2006 013 893     US-B1- 6 436 386**

• **HISASHI SESHIMA ET AL: "Control of bisphosphonate release using hydroxyapatite granules", JOURNAL OF BIOMEDICAL MATERIALS RESEARCH PART B: APPLIED BIOMATERIALS, vol. 78B, no. 2, August 2006 (2006-08), pages 215-221, XP055074932, ISSN: 1552-4973, DOI: 10.1002/jbm.b.30446**

**(Cont. next page)**

• KANDORI K ET AL: "PREPARATION AND CHARACTERIZATION OF SPHERICAL CALCIUM HIDROXYAPATITE", CHEMISTRY OF MATERIALS, AMERICAN CHEMICAL SOCIETY, WASHINGTON, US, vol. 7, no. 1, January 1995 (1995-01), pages 26-32, XP000513066, ISSN: 0897-4756, DOI: 10.1021/CM00049A007

Remarks:
  The file contains technical information submitted after the application was filed and not included in this specification

**Description**

**[Technical Field]**

The present invention relates to a calcium phosphate microsphere drug delivery system and a process for preparing the same. In particular, the present invention relates to a calcium phosphate microsphere drug delivery system for local delivery of a bisphosphonate drug, which is used for the treatment of osteoporosis, and a process for preparing the same.

**[Background Art]**

Bisphosphonate drugs, recently used for the prevention and treatment of osteoporosis, have been administered by oral route. The oral administration of bisphosphonate drugs has been reported to require an inconvenient regimen, namely, that patients should be treated for a long period of time (5 to 10 years), take them on an empty stomach, and stay in an upright position after taking them; the regimen also being accompanied by gastrointestinal disorders. In addition, due to their extremely low bioavailability of 1~2%, it is not expected that bisphosphonate drugs exert high therapeutic effects.

The oral bisphosphonate formulations have been shown to increase total body bone density, but are required to mainly act on fractures of the hip joint, spine, and wrist, which are susceptible to fractures. Thus, there is a need to develop a drug delivery system for local delivery of a bisphosphonate drug, which exhibits long-term therapeutic effects even though given once.

Calcium phosphate-based minerals are biocompatible materials being similar to bone mineral in terms of their components and crystallinity. When injected into the body, calcium phosphate-based minerals are easily resorbed by the surrounding cells, and dissociated into calcium ions ($Ca^{2+}$) and phosphate ions ($PO_4^{3-}$), thereby improving new bone formation when bone loss occurs. Owing to these advantages, calcium phosphate-based minerals have been largely studied for the development of drug delivery system. In particular, an antiosteoporotic agent, bisphosphonate is adsorbed and encapsulated onto the surface of calcium phosphate formulation, or the calcium phosphate formulation and a bisphosphonate drug are compressed into a tablet or powder, and then used for administration. If a drug delivery system developed by these methods is introduced into the bone loss-susceptible regions such as femur neck, distal radius, and vertebral bodies, the calcium phosphate formulation and drug show a bone strengthening effect by their synergistic effects.

However, there is no report about chemical association of the bisphosphonate drug and hydroxyapatite ($Ca_{10}(PO_4)_6(OH)_2$) that is one of the calcium phosphate-based minerals and comprises 70% of total body bone mineral content. Because of its low dissolution rate, pure hydroxyapatite is not preferred as an effective mineral for bone regeneration.

Seshima, H. et al. (2006), "Control of bisphosphonate release using hydroxyapatite granules", J. Biomed. Mater. Res. Part B: Appl Biomater, 78B: 215-221, disclose hydroxyapatite granules as carriers for local administration of bisphosphonates, wherein bisphosphonates are adsorbed to the granules.

When a drug is adsorbed onto the surface of conventional calcium phosphate formulation to prepare a drug delivery system, there are problems in that the drug loading amount is low and a complicated process is required.

To overcome such drawbacks, there is a need for the development of calcium phosphate formulation having excellent biocompatibility and an injectable drug delivery system that improves the resorption of the formulation and new bone formation and of f ers many advantages over the conventional oral drug delivery systems. Thus, many studies thereon have been made.

**[Disclosure]**

**[Technical Problem]**

Accordingly, the present inventors have made studies on injectable drug delivery systems capable of loading a large amount of drug and maximizing the therapeutic effects, which can be prepared by a simple process, in which an acidic aqueous solution containing hydroxyapatite, a bisphosphonate drug and urea is emulsified in oil phase, and calcium phosphate microspheres are crystallized by pH increase due to heat degradation of urea, and thus the bisphosphonate drug is encapsulated inside and on the surface of the calciumphosphate formulation as an in-situ reaction, so as to prepare a calcium phosphate microsphere drug delivery system. They found that the calcium phosphate microsphere drug delivery system can be prepared by using micro-sized spherical particles formed by emulsification, and thus is useful for local delivery of a bisphosphonate drug, thereby completing the present invention.

**[Technical Solution]**

It is an object of the present invention to provide a calcium phosphate microsphere drug delivery system for local delivery of a bisphosphonate drug, which is used for the treatment of osteoporosis, and a process for preparing the same.

**[Description of Drawings]**

FIG. 1 shows the result of scanning electron microscopy (SEM) of the alendronate-encapsulated calcium phosphate microsphere formulations according to the present invention, which are different from each other in the content of alendronate;

FIG. 2 shows the pH change due to heat degradation of urea in an in-situ reaction, which is performed for encapsulation of alendronate in the calcium phosphate microsphere formulation according to the present invention;

FIG. 3 shows the result of X-ray powder diffraction (XRD) analysis for the measurement of crystallinity of the alendronate-encapsulated calcium phosphate microsphere formulations according to the present invention, which are different from each other in the content of alendronate; and

FIG. 4 shows the result of measuring absorbance using a UV-VIS spectrophotometer to determine the content of alendronate encapsulated in each calcium phosphate microsphere formulation according to the present invention.

FIG. 5 shows that when the acidic aqueous solution containing urea is slowly heated from room temperature to 90°C urea hydrolysis occurs, causing the pH to increase from pH 2.5 to pH 6.5 in the acidic aqueous solution containing the calcium phosphate formulation and a bisphosphonate drug, alendronate.

**[Best Mode]**

The present invention provides a calcium phosphate microsphere drug delivery system, in which a bisphosphonate drug is encapsulated inside and on the surface of the hydroxyapatite.

Further, the present invention provides a process for preparing the calcium phosphate microsphere drug delivery system, comprising the steps of

1) adding an acidic aqueous solution (pH 2.5) containing hydroxyapatite ($Ca_{10}(PO_4)_6(OH)_2$), a bisphosphonate drug, and urea (($NH_2)_2CO$) to mineral oil to prepare a water-in-oil (W/O) emulsion, and

2) heating the water-in-oil emulsion prepared in step 1) to crystallize calcium phosphate microspheres by pH increase due to heat degradation of urea, and simultaneously to encapsulate the bisphosphonate drug inside and on the surface of the calcium phosphate formulation as an in-situ reaction.

Hereinafter, the present invention will be described in detail.

The bisphosphonate drug encapsulated in the calcium phosphate microsphere drug delivery system of the present invention is a useful drug for the treatment of osteoporosis. Examples thereof include etidronate, clodronate, palmidronate, alendronate, ibandronate, risedronate, zoledronate, tiludronate, YH529, icadronate, olpadronate, neridronate, and EB-1053, most preferably alendronate, but are not limited thereto.

The bisphosphonate drug is contained in an amount of 1~50% by weight, based on 100% by weight of hydroxyapatite. If the content of the bisphosphonate drug is more than 50% by weight, the drug amount to be encapsulated is too large, and thus interferes with recrystallization of calcium phosphate. Therefore, spherical calcium phosphate microspheres are hardly formed. If the content of the bisphosphonate drug is less than 1% by weight, the drug amount to be encapsulated is too little to achieve effective drug delivery.

In the process for preparing the calcium phosphate microsphere drug delivery system of the present invention, the first step of preparing a water-in-oil (W/O) emulsion is a step of making the acidic aqueous solution containing hydroxyapatite, the bisphosphonate drug and urea in a micro-sized form. That is, hydroxyapatite is dissolved in the acidic aqueous solution of pH 2.5, and then the bisphosphonate drug and urea are added thereto. The acidic aqueous solution is slowly added dropwise to mineral oil while stirring briskly, leading to emulsification, and thus the micro-sized aqueous solution exists uniformly in the mineral oil. During emulsification, a surfactant span85 is added to maintain the acidic aqueous solution in a micro-sized form without aggregation.

In the second step of the process for preparing the calcium phosphate microsphere drug delivery system of the present invention, the micro-sized acidic aqueous solution formed by the emulsification is subjected to in-situ reaction to prepare micro-sized spherical calcium phosphate particles, thereby encapsulating the bisphosphonate drug inside and on the surface of calcium phosphate formulation.

The pH increase caused by urea hydrolysis inside and on the surface of the heated acidic aqueous solution may account for the in-situ reaction mechanism encapsulating the bisphosphonate drug inside and on the surface of calcium phosphate formulation. The urea hydrolysis is represented by Reaction Scheme 1.

[Reaction Scheme 1]  $CO(NH_2)_2 + 3H_2O \rightarrow 2OH^- + 2NH_4^+ + CO_2$

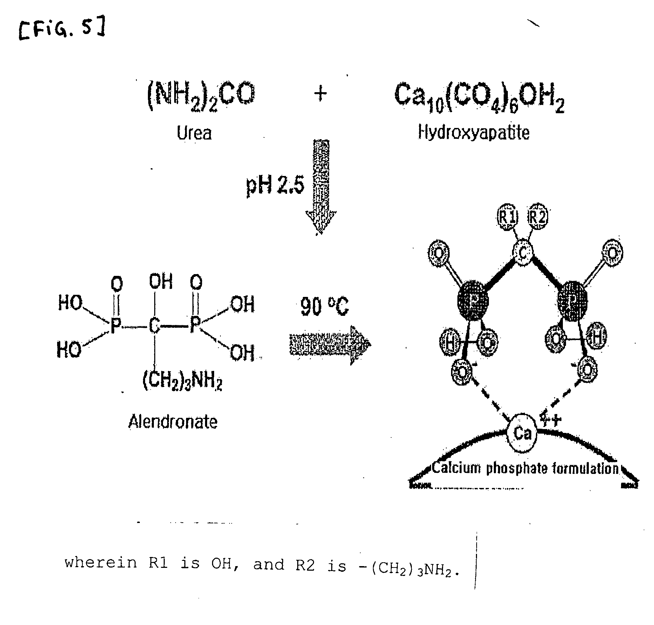

When the acidic aqueous solution containing urea is slowly heated from room temperature to 90°C urea hydrolysis occurs, causing the pH to increase from pH 2.5 to pH 6.5 in the acidic aqueous solution containing the calcium phosphate formulation and a bisphosphonate drug, alendronate, as shown in Fig. 5.

The pH increase in the acidic aqueous solution due to heat degradation of urea leads to recrystallization of hydroxyapatite into a new formulation of calcium phosphate. At this time, a strong binding affinity between the calcium ion ($Ca^{2+}$) and phosphate group of alendronate results in encapsulation of alendronate inside and on the surface of the micro-sized calcium phosphate formulation.

When the bisphosphonate drug is encapsulated inside and on the surface of hydroxyapatite according to the present invention, the micro-sized spherical calcium phosphate particles are uniformly formed. In XRD analysis, the formed calcium phosphate particles exhibit a different peak profile from pure hydroxyapatite. As a used amount of the bisphosphonate drug increases, crystallinity of the calcium phosphate microsphere formulation decreases, and a Ca/P molar ratio also decreases. Thus, it can be seen that the amount of the encapsulated drug gradually increases. The calcium phosphate microsphere formulation encapsulating the bisphosphonate drug of the present invention is therefore not pure hydroxyapatite but a new formulation of calcium phosphate.

The hydroxyapatite composition in the calcium phosphate formulation has a sufficiently low dissolution rate to ensure inefficient bone regeneration. By using the method of encapsulating a drug inside and on the surface of the calcium phosphate formulation according to the present invention, the hydroxyapatite composition in the calcium phosphate formulation can be changed, and dissolved into calcium ion ($Ca^{2+}$) and phosphate ion ($PO_4^{3-}$) when injected into the body, thereby promoting new bone generation when bone loss occurs. Furthermore, when an antiosteoporotic agent, bisphosphonate (in particular, alendronate) is encapsulated inside and on the surface of the newly formed calcium phosphate formulation according to the present invention, sustained release of alendronate from the calcium phosphate formulation may contribute to the bone strengthening effect in the regions expected to have low bone density.

**[Mode for Invention]**

Hereinafter, the preferred Examples are provided for better understanding. However, these Examples are for illustrative purposes only, and the invention is not intended to be limited by these Examples.

**Example 1: Preparation of alendronate-encapsulated calcium phosphate microsphere formulation**

**1. Formation of micro-sized acidic aqueous solution**

A micro-sized acidic aqueous solution was prepared by emulsification. In particular, 0.1 g of hydroxyapatite ($Ca_{10}(PO_4)_6(OH)_2$) was added to 20 ml of distilled water, and adjusted to pH 2.5 using hydrochloric acid (1 N HCl). A predetermined amount of alendronate ($C_4H_{18}NNaO_{10}P_2$) (11.1 mg, 25 mg, 42 mg) and 1 g of urea (($NH_2)_2CO$) were simultaneously added thereto, and the solution was mixed well at room temperature. 0.8 g of span85 was added to 79.2 g of mineral oil, and mixed well for 1 hr. The prepared acidic aqueous solution was slowly dropped to the mineral oil spinning at 1000 rpm, followed by spinning for 2 hrs to prepare the micro-sized acidic aqueous solution containing hydroxyapatite, alendronate and urea.

**2. Preparation of alendronate-encapsulated calcium phosphate microsphere formulation**

While spinning the mineral oil including the micro-sized acidic aqueous solution formed in the above procedure 1 at 1000 rpm, the temperature was slowly increased from room temperature to 90°C at a rate of 0.5°C per minute, and the mixture was left at 90°C for 7 hrs. After the reaction was completed, the mixture was centrifuged at 2500 rpm for 15 min, and the supernatant was discarded. Subsequently, the resultant was washed with ether using a centrifuge at 2500 rpm for 15 min three times, and then was freeze-dried for 48 hrs to obtain a calcium phosphate microsphere formulation encapsulating alendronate inside and on the surface thereof.

**Comparative Example 1: Preparation of calcium phosphate microsphere formulation encapsulating no alendronate**

A calcium phosphate microsphere formulation was prepared in the same manners as in Example 1, except for using no alendronate.

**Experimental Example 1: Measurement of size and shape of alendronate-encapsulated calcium phosphate microsphere formulation**

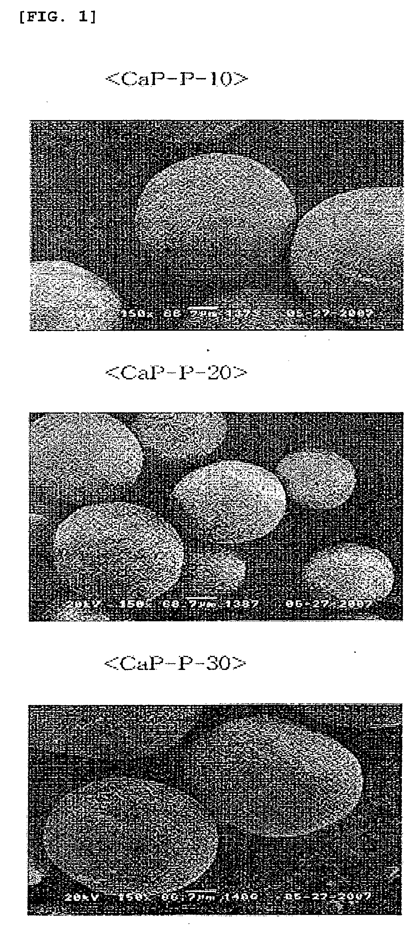

The alendronate-encapsulated calcium phosphate microsphere formulations, which were different from each other in the content of alendronate and prepared in Example 1, were coated with gold (Au) using a 108 auto sputter coater (Cressington Scientific Instruments), and then observed by scanning electron microscopy (SEM, SM-300, TOPCON). In this connection, scanning electron microscopy was performed at an accelerating voltage of 15 kV.
The results are shown in FIG. 1.
As shown in FIG. 1, the calcium phosphate microsphere formulations according to the present invention were found to have an average size of 200 $\mu$m, a narrow size distribution, and a spherical shape.

**Experimental Example 2 : Measurement of pH change due to heat degradation of urea during preparation of alendronate-encapsulated calcium phosphate microsphere formulation**

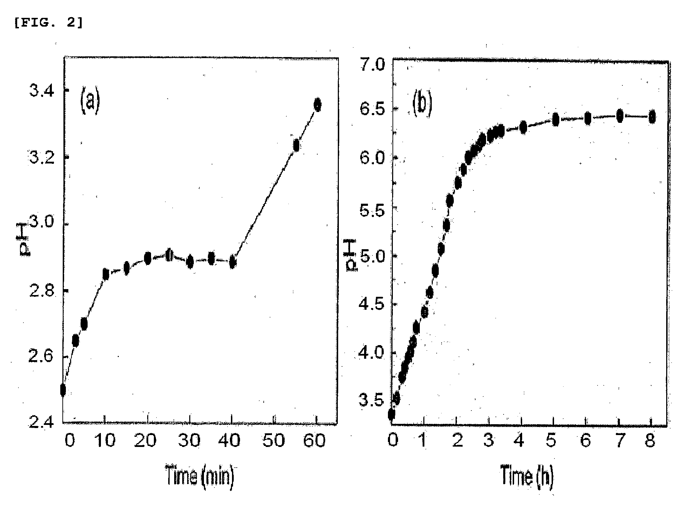

During in-situ reaction for the preparation of alendronate-encapsulated calcium phosphate microsphere formulation in Example 1, the pH change due to heat degradation of urea was measured.
The results are shown in FIG. 2.
As shown in FIG. 2, when the temperature was slowly increased from room temperature to 90 °C during in-situ reaction, a pH increase from 2.5 to 6.5 was observed, indicating that hydroxyapatite in the acidic aqueous solution was recrystallized into a new formulation of calcium phosphate.

**Experimental Example 3: Qualitative analysis of alendronate-encapsulated calcium phosphate microsphere formulation**

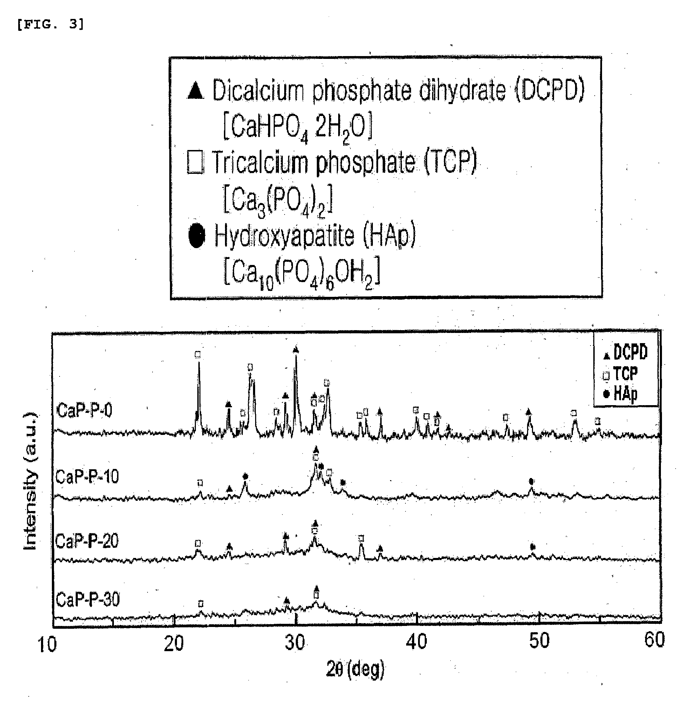

The alendronate-encapsulated calcium phosphate microsphere formulations, which were different from each other in the content of alendronate and prepared in Example 1, were subjected to X-ray powder diffraction (XRD) analysis using a Rigaku D/max_RB apparatus (Tokyo, Japan) to determine their crystallinity.
The results are shown in Fig. 3.
As shown in Fig. 3, the drug-encapsulated calcium phosphate microsphere formulations according to the present invention showed different diffraction peaks from pure hydroxyapatite. In addition, it was found that as the loading content of alendronate increased, the crystallinity of calcium phosphate microsphere formulation decreased.

**Experimental Example 4: Measurement of drug loading content and encapsulation efficiency of alendronate-encapsulated calcium phosphate microsphere formulation**

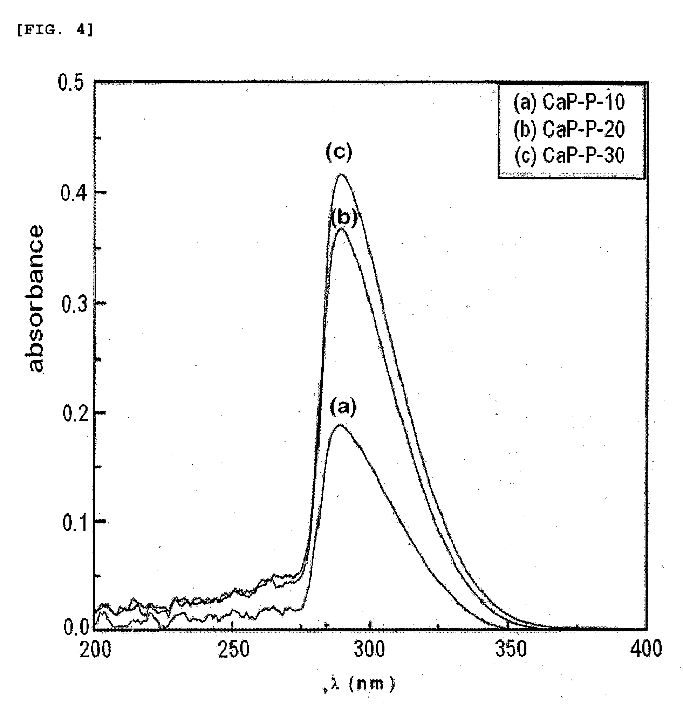

The following experiment was performed to measure drug loading content and encapsulation efficiency of alendronate-encapsulated calcium phosphate microsphere formulation according to the present invention.
To determine the content of alendronate encapsulated in the calcium phosphate microsphere prepared in Example 1, the alendronate-encapsulated microspheres were dissolved in an acidic solution, and iron (III) chloride was added thereto. Then, absorbance of the alendronate-iron (III) complex was determined using a UV-VIS spectrophotometer. The encapsulation efficiency of alendronate was calculated according to the following Mathematical Equation 1.
The results are shown in Table 1 and FIG. 4.

[Mathematical Equation 1]

Encapsulation efficiency of alendronate (% by weight) =

$(W_l/W_t) \times 100\%$

$W_l$: Weight of encapsulated alendronate
$W_t$: Weight of used alendronate

[Table 1]

| Sample (mg) | Hydroxyapatite (Hap)(g) | Alendronate (mg) | Drug-loading content (wt%) | Encapsulation efficiency(%) |
|---|---|---|---|---|
| CaP-P-0 | 0.1 | - | - | - |
| CaP-P-10 | 0.1 | 11.1 | 8.06 | 80.6 |
| CaP-P-20 | 0.1 | 25 | 14.44 | 72.2 |
| CaP-P-30 | 0.1 | 42 | 16.08 | 53.6 |

As shown in Table 1 and FIG. 4, the amount of alendronate that was encapsulated inside and on the surface of the calcium phosphate microsphere formulation of the present invention increased, as the used amount of alendronate increased. Prepared by using 11.1 mg of alendronate, the calcium phosphate microsphere formulation showed the most excellent encapsulation efficiency.

**Experimental Example 5: Measurement of Ca/P ratio in alendronate-encapsulated calcium phosphate microsphere formulation**

A Ca/P molar ratio was measured by ICP-MS using a Perkin Elmer Sciex Elan 6100 with respect to the alendronate-encapsulated calcium phosphate microsphere formulations, which were different from each other in the content of alendronate and prepared in Example 1, the calcium phosphate microsphere formulation encapsulating no alendronate which were prepared in Comparative Example 1, and pure hydroxyapatite.

The results are shown in Table 2.

[Table 2]

| Sample | Ca/P molar ratio |
|---|---|
| HAp | 1.67 |
| CaP-P-0 | 1.56 |
| CaP-P-10 | 1.35 |
| CaP-P-20 | 1.24 |
| CaP-P-30 | 1.21 |

As shown in Table 2, unlike the Ca/P molar ratio of pure hydroxyapatite (1.67), that of the alendronate-encapsulated calcium phosphate microsphere of the present invention decreased, as the content of alendronate increased, indicating that the amount of encapsulated drug gradually increased. Thus, it can be seen that the alendronate-encapsulated calcium phosphate microsphere formulation of the present invention is not pure hydroxyapatite but a new formulation of calcium phosphate.

**[Industrial Applicability]**

According to the present invention, an alendronate-encapsulated calcium phosphate microsphere drug delivery system can be prepared by a simple process, in which an acidic aqueous solution containing hydroxyapatite, a bisphosphonate drug and urea is emulsified in oil phase, and calcium phosphate microspheres are crystallized by pH increase due to heat degradation of urea, and thus the bisphosphonate drug is simultaneously encapsulated inside and on the surface of the calcium phosphate formulation as an in-situ reaction, so as to prepare the calcium phosphate microsphere drug delivery system capable of loading a larger amount of drug, compared to the conventional surface adsorption method.

**Claims**

1.    A calcium phosphate microsphere drug delivery system encapsulating a bisphosphonate drug inside and on the surface of hydroxyapatite.

2. The calcium phosphate microsphere drug delivery system according to claim 1, wherein the bisphosphonate drug is one selected from the group consisting of etidronate, clodronate, palmidronate, alendronate, ibandronate, risedronate, zoledronate, tiludronate, YH529, icadronate, olpadronate, neridronate, and EB-1053.

3. The calcium phosphate microsphere drug delivery system according to claim 2, wherein the bisphosphonate drug is alendronate.

4. The calcium phosphate microsphere drug delivery system according to any one of claims 1 to 3, wherein the bisphosphonate drug is contained in an amount of 1-50% by weight, based on 100% by weight of hydroxyapatite.

5. A process for preparing a calcium phosphate microsphere drug delivery system, comprising the steps of

   1) adding an acidic aqueous solution (pH 2.5) containing hydroxyapatite ($Ca_{10}(PO_4)_6(OH)_2$), a bisphosphonate drug, and urea (($NH_2)_2CO$) to mineral oil to prepare a water-in-oil (W/O) emulsion, and
   2) heating the water-in-oil emulsion prepared in step 1) to crystallize calcium phosphate microspheres by pH increase due to heat degradation of urea, and simultaneously to encapsulate the bisphosphonate drug inside and on the surface of the calcium phosphate formulation as an in-situ reaction.

6. The process for preparing a calcium phosphate microsphere drug delivery system according to claim 5, wherein the water-in-oil emulsion is heated from room temperature to 90°C

7. The process for preparing a calcium phosphate microsphere drug delivery system according to claim 5, wherein the pH increase is from 2.5 to 6.5.

**Patentansprüche**

1. Ein Kalziumphosphat-Mikrokügelchen-Arzneimittelabgabesystem, das ein Bisphosphonatmedikament im Inneren und auf der Oberfläche von Hydroxyapatit einkapselt.

2. Das Kalziumphosphat-Mikrokügelchen-Arzneimittelabgabesystem nach Anspruch 1, wobei das Bisphosphonatmedikament eines ist, das ausgewählt ist aus der Gruppe bestehend aus Etidronat, Clodronat, Palmidronat, Alendronat, Ibandronat, Risedronat, Zoledronat, Tiludronat, YH529, Icadronat, Olpadronat, Neridronat und EB-1053.

3. Das Kalziumphosphat-Mikrokügelchen-Arzneimittelabgabesystem nach Anspruch 2, wobei das Bisphosphonatmedikament Alendronat ist.

4. Das Kalziumphosphat-Mikrokügelchen-Arzneimittelabgabesystem nach irgendeinem der Ansprüche 1 - 3, wobei das Bisphosphonatmedikament in einer Menge von 1-50 Gew%, basierend auf 100 Gew% von Hydroxyapatit, enthalten ist.

5. Ein Verfahren zur Herstellung eines Kalziumphosphat-Mikrokügelchen-Arzneimittelabgabesystems, umfassend die Schritte

   1) Zugeben einer sauren wässrigen Lösung (pH 2,5), die Hydroxyapatit ($Ca_{10}(PO_4)_6(OH)_2$), ein Bisphosphonatmedikament und Harnstoff (($NH_2)_2CO$) enthält, zu Mineralöl, um eine Wasser-in-Öl (W/O) Emulsion herzustellen
   und
   2) Erhitzen der in Schritt 1) hergestellten Wasser-in-Öl Emulsion, um Kalziumphosphat-Mikrokügelchen durch pH-Steigerung durch Hitzeabbau von Harnstoff zu kristallisieren, und gleichzeitig das Bisphosphonatmedikament im Inneren und auf der Oberfläche der Kalziumphosphat-Formulierung als eine in-situ Reaktion einzukapseln.

6. Das Verfahren zur Herstellung eines Kalziumphosphat-Mikrokügelchen-Arzneimittelabgabesystems nach Anspruch 5, wobei die Wasser-in-Öl Emulsion von Raumtemperatur auf 90 °C erhitzt wird.

7. Das Verfahren zur Herstellung eines Kalziumphosphat-Mikrokügelchen-Arzneimittelabgabesystems nach Anspruch 5, wobei der pH von 2,5 auf 6,5 gesteigert wird.

**Revendications**

1. Système d'administration de médicaments sous forme de microsphères de phosphate de calcium encapsulant un médicament biphosphonate à l'intérieur et sur la surface d'hydroxyapatite.

2. Système d'administration de médicaments sous forme de microsphères de phosphate de calcium selon la revendication 1, où le médicament biphosphonate est l'un choisi dans le groupe constitué d'étidronate, clodronate, palmidronate, alendronate, ibandronate, risédronate, zolédronate, tiludronate, YH529, icadronate, olpadronate, néridronate, et EB-1053.

3. Système d'administration de médicaments sous forme de microsphères de phosphate de calcium selon la revendication 2, où le médicament biphosphonate est l'alendronate.

4. Système d'administration de médicaments sous forme de microsphères de phosphate de calcium selon l'une quelconque des revendications 1 à 3, où le médicament biphosphonate est contenu dans une quantité de 1 à 50 % en poids, en se basant sur 100 % en poids d'hydroxyapatite.

5. Procédé de préparation d'un système d'administration de médicaments sous forme de microsphères de phosphate de calcium, comprenant les étapes suivantes

   1) l'addition d'une solution aqueuse acide (pH 2,5) contenant de l'hydroxyapatite ($Ca_{10}(PO_4)_6(OH)_2$), un médicament biphosphonate, et de l'urée (($NH_2)_2CO$) à une huile minérale pour préparer une émulsion eau dans l'huile (E/H), et
   2) le chauffage de l'émulsion eau dans l'huile préparée dans l'étape 1) pour cristalliser des microsphères de phosphate de calcium par une augmentation du pH due à la dégradation par la chaleur de l'urée, et pour encapsuler simultanément le médicament biphosphonate à l'intérieur et sur la surface de la formulation de phosphate de calcium en tant que réaction *in situ.*

6. Procédé de préparation d'un système d'administration de médicaments sous forme de microsphères de phosphate de calcium selon la revendication 5, dans lequel l'émulsion eau dans l'huile est chauffée de la température ambiante à 90 °C.

7. Procédé de préparation d'un système d'administration de médicaments sous forme de microsphères de phosphate de calcium selon la revendication 5, dans lequel l'augmentation de pH est de 2,5 à 6,5.

[FIG. 1]

<CaP-P-10>

<CaP-P-20>

<CaP-P-30>

[FIG. 2]

[FIG. 3]

[FIG. 4]

[FIG. 5]

$(NH_2)_2CO$ + $Ca_{10}(CO_4)_6OH_2$
Urea          Hydroxyapatite

pH 2.5

Alendronate

90 °C

Calcium phosphate formulation

wherein R1 is OH, and R2 is $-(CH_2)_3NH_2$.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SESHIMA, H. et al.** Control of bisphosphonate release using hydroxyapatite granules. *J. Biomed. Mater. Res. Part B: Appl Biomater,* 2006, vol. 78B, 215-221